# EUROPEAN PATENT APPLICATION

(11) **EP 4 589 168 A1**
(43) Date of publication of application: **23.07.2025**
(21) Application number: 24152836.3
(22) Date of filing: 19.01.2024
(51) Int. Cl.: F16G 9/00, F16D 49/00, F16C 1/10

(54) **SYSTEM, ASSISTING DEVICE, AND METHOD OF OPERATING A SYSTEM**

(71) Applicant: Friedrich-Alexander-Universität Erlangen-Nürnberg, 91054 Erlangen (DE)
(72) Inventor: WALTER, Jonas, 91058 Erlangen (DE); BRAUN, Dominik, 91058 Erlangen (DE); FRANKE, Jörg, 91080 Marolffstein (DE); DEL VECCHIO, Alessandro, 91052 Erlangen (DE); SIMPETRU, Raul, 91080 Marloffstein (DE); WEBER, Nico, 91052 Erlangen (DE)
(74) Representative: Vossius & Partner Patentanwälte Rechtsanwälte mbB

(57) **Abstract**

The present invention relates to a system (10) for transmitting power. The system (10) comprises a first component (12) which includes a first cable winch (14) configured to rotate to at least partially wind and unwind a first cable (16) on the first cable winch (14). The system (10) comprises a second component (20) which includes a second cable winch (22) configured to rotate to at least partially wind and unwind a second cable (24) on the second cable winch (22). The first component (14) comprises a first engagement device (26) and the second component (20) comprises a second engagement device (30) configured to selectively engage with the first engagement device (20) to couple and decouple the first cable winch (14) and the second cable winch (22) to and from each other, respectively, such that rotation of one of the first cable winch (14) and the second cable winch (22) causes rotation of the other of the first cable winch (14) and the second cable winch (22), when the first cable winch (14) and the second cable winch (22) are in a coupled state.

The present invention further relates to an assisting device (90) and a method of operating a system (10).

## Description

A transmission of power between two components, e.g., a source of power, e.g., a motor, and a component to which the power is to be applied, is employed in a variety of fields and/or for a variety of different purposes. In many cases, a cable is employed as a means for transmitting power between two or more components, or at least to assist in transmitting power between two or more components.

However, there are several disadvantages in the systems for transmitting power known from the prior art, in particular which involve a cable for transmitting power, which haven't been addressed, or at least haven't sufficiently been addressed.

For instance, it may be necessary to disassemble and/or detach, in particular temporarily, one or more components of the system, for instance the cable which is configured to transmit the power and/or a motor provided in the system. This may be relatively laborious and time-consuming, in particular when the one or more components should be reattached to the system.

Moreover, the length of the cable employed in the system may vary during use and/or depending on the application. For this purpose, the cable may be wound und unwound on a winding device, e.g., a cable winch, in order to reduce or increase the length of the cable. However, several disadvantages remain in the systems known from the prior art in this regard. For instance, in particular in view of the above-described desire and/or need to temporarily detach and reattach the cable in the respective system, the prior art has not solved the above-discussed aspects, at least not sufficiently.

It is therefore an object of the present invention to provide an improved system for transmitting power, in particular by at least partially improving one or more of the above-identified disadvantages.

This object is achieved by a system for transmitting power, as defined by the features of claim 1. Variations and further developments are defined by the features of the dependent claims.

The system may comprise a first component which may include a first cable winch which may be configured to rotate to at least partially wind and unwind a first cable on the first cable winch. The system may comprise a second component which may include a second cable winch which may be configured to rotate to at least partially wind and unwind a second cable on the second cable winch. The first component may comprise a first engagement device and the second component may comprise a second engagement device which may be configured to selectively engage with the first engagement device to couple and decouple, i.e., operatively couple and decouple, the first cable winch and the second cable winch to and from each other, respectively, in particular such that rotation of one of the first cable winch and the second cable winch causes rotation of the other of the first cable winch and the second cable winch, when the first cable winch and the second cable winch are in a coupled state. In other words, engagement between the first engagement device and the second engagement device allows movement and/or forces and/or torque of the first cable winch to be transferred and/or translated to the second cable winch and/or vice versa. In other words, rotation of the first cable winch may rotatably drive the second cable winch and/or vice versa. This may allow movement and/or forces and/or torque of the first cable to be transferred and/or translated to the second cable and/or vice versa.

The first cable and the second cable are separate cables. However, coupling the first cable winch and the second cable winch may allow the first cable and the second cable to be connected/coupled to act and be applied as a single and/or continuous cable.

The system described herein may provide an efficient and relatively simple means for selectively, i.e., upon demand, coupling and decoupling at least two cables, i.e., the first cable and the second cable, to and from each other, respectively, in particular by coupling the first cable winch and the second cable winch by engaging the first engagement device with the second engagement device and decoupling the first cable winch and the second cable winch by disengaging the first engagement device from the second engagement device, respectively. For instance, this may allow the entire first cable winch and/or second cable winch to be removed from the system. This may allow, e.g., at least one of the cables to be serviced and/or adjusted, in particular while one the cables remains in place. This may reduce the efforts for maintenance and/or servicing and/or performing adjustments and/or corrections to the system. Moreover, the system may provide a relatively compact construction.

Moreover, configuring the system with at least two cable winches, i.e., the first cable winch and the second cable winch, may allow a greater volume of cable, e.g., a greater length of cable, to be wound and unwound onto the cable winches and/or may reduce the size and/or weight of each cable winch, compared with a single cable winch.

Moreover, the inventors have discovered that connecting the first cable and the second cable by coupling the first cable winch and the second cable winch has several further advantages. For instance, this may allow a relatively high power and/or relatively high forces and/or relatively high torque to be transferred between the first cable and the second cable. Moreover, this may provide a relatively reliable connection/coupling between the first cable and the second cable. In particular, any potential disadvantages, e.g., in the amount of power and/or torque which may be transferred between the first cable and the second cable and/or in the efficiency thereof, compared with a single continuous cable, may be minimized or eliminated entirely with the system disclosed herein.

The first cable and the second cable may extend in substantially opposite directions from the first component and the second component, respectively, when the first cable winch and the second cable winch are in a coupled state.

The first component may be configured to at least partially house the first cable and/or the second component may be configured to at least partially house the second cable.

The first component may include a housing configured to at least partially house the first cable winch. Alternatively, or additionally, the second component may include a housing configured to at least partially house the second cable winch. The housing of the first component may include the first engagement device. In other words, the first engagement device may be defined in or on the housing of the first component. Alternatively, or additionally, the housing of the second component may include the second engagement device. In other words, the second engagement device may be defined in or on the housing of the second component.

The housing of the first component and the housing of the second component may each be configured as a partial housing, e.g., as a half shell, such that the housing of the first component and the housing of the second component may be combined to an outer housing which at least partially encompasses the first cable winch and the second cable winch, when the first component and the second component are coupled and/or secured to each, e.g., by the first engagement device and the second engagement device and/or a securing device, which is described further below. For instance, the housing of the first component and the housing of the second component may each be configured as a half shell. Each partial housing may be open on a first side of the partial housing, e.g., each partial housing may be configured such that at least a portion of the respective cable winch is exposed, and a second side of the partial housing may be at least partially, preferably substantially completely, closed with respect to an environment. The exposed first sides of the partially housings may be mated to each other to engage the first engagement deice with the second engagement device in order to couple the first cable winch and the second cable winch.

The first cable winch and/or the second cable winch may be configured such that at least one winding, preferably a plurality of windings, of the first cable and the second cable, respectively, is windable onto and unwindable from the first cable winch and the second cable winch, respectively.

The first component and the second component may be configured as a clutch, wherein the first component is a first member of the clutch and the second component is a second member of the clutch. The clutch may be configured to be disengaged by decoupling the first component from the second component and/or vice versa.

The system may be configured such that, when the first cable winch and the second cable winch are in a coupled state, rotation of the first cable winch rotatably drives the second cable winch such that:
the first cable is wound onto the first cable winch and the second cable is unwound from the second cable winch;
   and/or
the first cable is unwound from the first cable winch and the second cable is wound onto the second cable winch. This may allow the length of the first cable or the second cable, i.e., the effective length of the respective cable which is not wound on the respective cable winch, to be increased, while the length of the other of the first cable and the second cable is decreased. This may allow tensile forces to be transmitted between the first cable and the second cable, while limiting the total effective length of the first cable and second cable or maintaining a substantially constant total effective length of the first cable and second cable.

Alternatively, or additionally, the system may be configured such that, when the first cable winch and the second cable winch are in a coupled state, rotation of the second cable winch rotatably drives the first cable winch such that:
the first cable is wound onto the first cable winch and the second cable is unwound from the second cable winch;
   and/or
the first cable is unwound from the first cable winch and the second cable is wound onto the second cable winch.

In other words, the rotation of the first cable winch and the second cable winch may be initiated by the first cable winch and/or the second cable winch.

The system may be configured such that a winding axis of the first cable winch, about which the first cable is windable and/or unwindable, and a winding axis of the second cable winch, about which the second cable is windable and/or unwindable, substantially coincide. This may allow a relatively direct transmission of forces and/or movement, in particular rotation, between the first cable winch and the second cable winch. Moreover, this may provide a relatively compact configuration, e.g., by arranging the first cable winch and the second cable winch side by side, i.e., in series along the winding axis of the first cable winch and the winding axis of the second cable winch. This may also increase the sturdiness and robustness of the system, in particular by generating and/or transmitting a moment/torque about a single and/or common axis.

Alternatively, the winding axis of the first cable winch and the winding axis of the second cable winch may extend substantially parallel to each.

The first cable winch may be configured to be biased towards winding the first cable onto the first cable winch. Alternatively, or additionally, the second cable winch may be configured to be biased towards winding the second cable onto the second cable winch. This may allow tension to be maintained on the first cable and/or the second cable, or facilitate maintaining tension on the first cable and/or the second cable, and/or may reduce slack in the first cable and/or the second cable.

The system may include at least one motor, preferably at least one electric motor, which is coupled, or configured to be coupled, to the first cable and/or the second cable to drive the first cable and/or the second cable, respectively.

The first cable winch and/or the second cable winch may include at least one rotary bearing, preferably at least one rolling bearing, preferably at least one ball bearing, to allow the first cable winch and/or the second cable winch, respectively, to rotate about an axis, e.g., a winding axis.

The first engagement device may be a first toothed gearing, preferably a first toothed gearwheel, and/or the second engagement device may be a second toothed gearing, preferably a second toothed gearwheel. Thus, one or more teeth of the first toothed gearing may engage with one or more teeth of the second toothed gearing to operatively couple and decouple the first cable winch and the second cable winch to and from each other, respectively, such that rotational movement may be transferred between the first cable winch and the second cable winch via the engaged teeth, when the first cable winch and the second cable winch are in a coupled state. This may allow relatively high forces and/or a relatively high torque to be transferred between the first cable winch and the second cable winch. Moreover, this may also allow a transmission ratio between the first cable winch and the second cable winch to be adjusted and/or set and/or varied, e.g., a ratio other than 1:1, or at least facilitate adjusting and/or setting a transmission ratio.

Alternatively, different mechanisms may be employed for coupling and decoupling the first cable winch and the second cable winch to and from each other. For instance, the first engagement device and the second engagement device may be configured as a friction clutch, in particular a rotational friction clutch, i.e., such that the first engagement device is configured to frictionally engage the second engagement device such that rotational movement may be transferred between the first cable winch and the second cable winch via friction, in particular only friction, between the first engagement device and the second engagement device. Further alternatively, the first engagement device and the second engagement device may be configured as a tongue and groove connection.

The first toothed gearing and the second toothed gearing may each configured as a face gearing or a bevel gearing. This may further increase the force(s) and/or torque(s) which can be transferred between the first cable winch and the second cable winch. Moreover, this may also facilitate the coupling process for coupling the first cable winch to the second cable winch via the face gearing or the bevel gearing, e.g., compared with a gearing with axially extending teeth. This may also reduce the axial length of the coupled first cable winch and second cable winch, in particular along a winding axis of the first cable winch and/or second cable winch. A face gearing, within the context of the present disclosure, is understood as a gearing with a plurality of face splines, e.g., which are arranged on an end face of the respective gearing.

The system may include at least one Bowden cable. The Bowden cable may include at least one cable sheath in which the first cable and/or the second cable is/are guidable such that the first cable and/or the second cable is/are moveable within the cable sheath, relative to the sheath. This may allow tensile forces and compression forces, or pushing forces, to be transferred via the first cable and/or the second cable, with the assistance of the Bowden cable.

The Bowden cable may be configured to be connected to a component, e.g., a third component, e.g., a section of an orthosis and/or prosthesis, to which one or more forces are to be applied.

The cable sheath may be substantially non-extensible and/or non-compressible, at least along a longitudinal direction of the cable sheath.

The system may include at least one securing device configured to fixedly secure the first component to the second component to maintain the first cable winch and the second cable winch in a coupled state. The securing device may include at least one first securing element provided and/or defined on the first component and at least one second securing element provided and/or defined on the second component. The first securing element and the second securing element may be configured to releasably engage each other to fixedly secure the first component to the second component. For instance, the first securing element and/or the second securing element may be configured as a clip and/or latch configured to engage the other of first securing element and the second securing element. The first securing element and the second securing element may be configured to releasably engage in a frictional and/or form-fit manner.

The securing device may be configured as a quick-release mechanism. A quick-release mechanism, within the context of the present disclosure, is understood as a mechanism which allows the securing device to be released easily and quickly, without the use of tools, e.g., by simply pulling the first component and the second component apart and/or by simply rotating the first component and the second component relative to each other and/or by simply pushing a release actuator, e.g., a section of the securing device, e.g., to remove the first component and/or the second component. This may facilitate removing the first component and/or the second component from the system.

The securing device may be configured as a snap-fit connection, a latching connection, a screw connection, and/or a magnetic connection.

The system may comprise at least one stop device configured to, preferably selectively, block and/or limit rotation of the first cable winch and/or the second cable winch. This may allow a length of the first cable and/or a length of the second cable to be maintained substantially constant, or at least within a limited range, in particular when one of the first cable winch and the second cable winch is detached from the system. There is a risk that the length of the first cable and/or the length of the second cable may be unintentionally changed, when the first component and/or the second component is/are detached and/or removed from the system, e.g., by unintentionally pulling the first cable and/or the second cable and/or unintentionally rotating the first cable winch and/or the second cable winch. Thus, blocking and/or limiting rotation of the first cable winch and/or the second cable winch by means of the stop device may facilitate operation of the system, since the length of the first cable and/or the length of the second cable may not have to be readjusted, or only has to be minimally readjusted, after the first component and/or the second component has/have been reattached.

Additionally, or alternatively, the stop device may serve as a safety mechanism, e.g., to lock the first cable winch and/or the second cable winch, e.g., in case of an emergency and/or a hazard.

The stop device may be configured to be operated, in particular activated, automatically, preferably when the first cable winch and the second cable winch are decoupled from each other. This may further facilitate the operation of the system and/or may increase the effectiveness of the stop device and/or may increase the user-friendliness of the system. Alternatively, or additionally, the stop device may be configured to be operated, in particular activated, manually.

The term "automatically", within the context of the present disclosure, is understood as an action, e.g., an activation and/or deactivation, e.g., of the stop device, which is not directly performed by a user intervention and/or wherein the user intervention which triggers and/or results in the "automatic action" does not have the sole purpose of performing the "automatic action". In other words: the automatic action may be a secondary effect of the respective user intervention. An event and/or user intervention, e.g., manual coupling of the first cable winch and the second cable winch by a user, may trigger the "automatic" action, e.g., an activation and/or deactivation of the stop device.

The stop device may be configured to engage with the first engagement device and/or the second engagement device, e.g., with one or more teeth of the first engagement device and/or the second engagement device.

The stop device may be configured to be deactivated automatically, preferably when the first cable winch and the second cable winch are coupled to each other, and/or manually. This may further facilitate the operation of the system and/or may increase the user-friendliness of the system.

The stop device may include one or more teeth configured to engage one or more teeth of the first cable winch and/or one or more teeth of the second cable winch. For instance, the first cable winch may include a gearwheel, or a segment of a gearwheel, which includes one or more teeth arranged on an outer circumference of the gearwheel. Alternatively, or additionally, the second cable winch may include a gearwheel, or a segment of a gearwheel, which includes one or more teeth arranged on an outer circumference of the gearwheel. The one or more teeth of the stop device may be configured to engage the one or more teeth of the gearwheel of the first cable winch and/or the one or more teeth of the gearwheel of the second cable winch. The engagement between the teeth of the stop device and the teeth of the first cable winch and/or the second cable winch may block and/or limit rotation of the first cable winch and/or the second cable winch.

The stop device may be movable between an engaged position, in which the stop device blocks and/or limits rotation of the first cable winch and/or the second cable winch, and a disengaged position, in which the stop device does not block and/or limit rotation of the first cable winch and/or the second cable winch.

The stop device may be biased towards the engaged position in which the stop device blocks and/or limits rotation of the first cable winch and/or the second cable winch. The bias, e.g., one or more biasing forces toward the engaged position, may be provided, e.g., by at least one spring element. For instance, at least one spring element, e.g., spring steel sheet, may be provided. Alternatively, or additionally, the stop device may be shaped and/or formed and/or dimensioned such that the stop device may be biased towards the engaged position, i.e., such that the stop device is in the engaged position, in the absence of one or more counter-forces.

The stop device may be configured such that at least a portion of the stop device is deflected away from the engaged position, when the first cable winch and the second cable winch are coupled to each other, to a disengaged position, in which the stop device does not block and/or limit rotation of the first cable winch and/or the second cable winch. For instance, the system may be configured such that, while the first cable winch and the second cable winch are coupled to each other and/or when the first cable winch and the second cable winch have been coupled to each other, a section of the first component and/or the second component may contact and/or engage with at least a section of the stop device to move the stop device away from the engage position, such that the first cable winch and/or the second cable winch can rotate freely, i.e., without being blocked and/or limited by the stop device.

The first cable winch and/or the second cable winch may include a cable receiving portion configured to receive at least a section of the first cable and/or the second cable, respectively, by winding the respective cable at least partially about the cable receiving portion. The cable receiving portion may be arranged between the respective engagement device and a section of the respective cable winch configured to be engaged by the stop device. The cable receiving portion, the respective engagement device, and the section of the respective cable winch configured to be engaged by the stop device, which may include one or more teeth as described above, may be configured as a single coherent unit. This may provide a relatively compact system and/or cable winch.

The first component and the second component may be mirror-symmetrical.

The present disclosure also relates to an assisting device configured to be worn by a user and to assist movement of the user. The assisting device may include at least one system according to any of the embodiments described herein to transmit power from at least one power source, in particular at least one electric motor, to the assisting device.

The assisting device may be configured as an orthosis, in particular a hand orthosis, or a prosthesis, in particular a hand prosthesis.

The hand orthosis or hand prosthesis may include at least two finger elements which may be at least partially flexible and/or manipulatable, in particular by means of at least one joint of the finger elements. The finger elements may be coupled or couplable to the first cable and/or the second cable of the system to manipulate, preferably drive, the finger elements via the first cable and/or the second cable.

The assisting device may be configured to be adjustable in size.

The assisting device may include at least two magnets configured to fixate the assisting device on the user's body.

The assisting device may include at least one connecting device, in particular at least one snap fastener and/or at least one Velcro fastener, configured to fixate the assisting device to the user's body.

The assisting device may include the at least one power source.

The present disclosure also relates to a robot configured to be manipulated about and/or along one or more axes. The robot may include at least one system according to any of the embodiments described herein to transmit power from at least one power source, in particular at least one electric motor, in particular from and/or to and/or within the robot.

The above-described objects, i.e., the assisting device and the robot, are examples for an application and/or implementation of the system for transmitting power as described herein. However, the system may be applied and/or implemented in a variety of other objects. In general, the system may be applied and/or implemented in any object in which a transmission of power is required and/or desired. For instance, the system may be applied and/or implemented in a transportation device and/or a vehicle, e.g., a bicycle.

A further object of the present disclosure is to provide an improved method of operating a system for transmitting power, in particular by at least partially improving one or more of the disadvantages mentioned at the beginning. The features, embodiments, and advantages, as detailed herein with respect to the system, apply to the method accordingly.

The system, which is operated via the method, may comprise a first component which may include a first cable winch, which may be configured to rotate to at least partially wind and unwind a first cable on the first cable winch, and a first engagement device. The system may include a second component which includes a second cable winch, which may be configured to rotate to at least partially wind and unwind a second cable on the second cable winch, and a second engagement device configured to selectively engage with the first engagement device.

The method may comprise:
engaging the first engagement device with the second engagement device to couple the first cable winch and the second cable winch to each other such that rotation of one of the first cable winch and the second cable winch causes rotation of the other of the first cable winch and the second cable winch, when the first cable winch and the second cable winch are in a coupled state;
   and/or
disengaging the first engagement device from the second engagement device to decouple the first cable winch from the second cable winch.

The method may comprise:
activating at least one stop device by moving the stop device to an engaged position to block and/or limit rotation of the first cable winch and/or the second cable winch by the stop device.

The stop device may be moved automatically to the engaged position when the first cable winch and the second cable winch are decoupled from each other.

The method may comprise:
deactivating the stop device by moving the stop device from the engaged position to a disengaged position, in which the stop device does not block and/or limit rotation of the first cable winch and/or the second cable winch, preferably automatically, preferably when the first cable winch and the second cable winch are coupled to each other.

The following list of aspects provides preferred embodiments of the present disclosure:
1. System for transmitting power, the system comprising:
   a first component configured to at least partially receive and/or engage with a first transmitting device, preferably a first cable, preferably wherein the first component includes a first cable winch configured to rotate to at least partially wind and unwind the first cable on the first cable winch;
   a second component configured to at least partially receive and/or engage with a second transmitting device, preferably a second cable, preferably wherein the second component includes a second cable winch configured to rotate to at least partially wind and unwind the second cable on the second cable winch;
   wherein the first component comprises a first engagement device and the second component comprises a second engagement device configured to selectively engage with the first engagement device to couple and decouple the first cable, preferably the first cable winch, and the second cable, preferably the second cable winch, to and from each other, respectively, preferably such that rotation of one of the first cable winch and the second cable winch causes rotation of the other of the first cable winch and the second cable winch, preferably when the first cable winch and the second cable winch are in a coupled state.
2. System according to aspect 1, wherein the system is configured such that rotation of the first cable winch rotatably drives the second cable winch and/or wherein rotation of the second cable winch rotatably drives the first cable winch, when the first cable winch and the second cable winch are in a coupled state.
3. System according to aspect 1 or 2, wherein the system is configured such that, when the first cable winch and the second cable winch are in a coupled state:
   rotation of the first cable winch rotatably drives the second cable winch such that the first cable is wound onto the first cable winch and the second cable is unwound from the second cable winch and/or the first cable is unwound from the first cable winch and the second cable is wound onto the second cable winch;
      and/or
   rotation of the second cable winch rotatably drives the first cable winch such that the first cable is wound onto the first cable winch and the second cable is unwound from the second cable winch and/or the first cable is unwound from the first cable winch and the second cable is wound onto the second cable winch.
4. System according to any one of the preceding aspects, wherein a winding axis of the first cable winch and a winding axis of the second cable winch coincide and/or extend parallel to each other.
5. System according to any one of the preceding aspects, wherein the first cable winch is biased towards winding the first cable onto the first cable winch and/or the second cable winch is biased towards winding the second cable onto the second cable winch.
6. System according to any one of the preceding aspects, further including at least one motor which is coupled to the first cable and/or the second cable to drive the first cable and/or the second cable, respectively.
7. System according to any one of the preceding aspects, wherein the first engagement device is configured as a first toothed gearing and the second engagement device is configured as a second toothed gearing.
8. System according to aspect 7, wherein the first toothed gearing and the second toothed gearing are each configured as a face gearing or a bevel gearing.
9. System according to any one of the preceding aspects, wherein the first component is configured to at least partially house the first cable and/or the second component is configured to at least partially house the second cable.
10. System according to any one of the preceding aspects, further including at least one Bowden cable which includes at least one cable sheath in which the first cable and/or the second cable is/are guidable such that the first cable and/or the second cable is/are moveable relative to the at least one cable sheath.
11. System according to any one of the preceding aspects, wherein the first cable and the second cable extend in substantially opposite directions from the first component and the second component, respectively, when the first cable winch and the second cable winch are in a coupled state.
12. System according to any one of the preceding aspects, further including at least one securing device configured to fixedly secure the first component to the second component to maintain the first cable winch and the second cable winch in a coupled state.
13. System according to aspect 12, wherein the securing device is configured as a quick-release mechanism.
14. System according to aspect 12 or 13, wherein the securing device is configured as a snap-fit connection, a screw connection, and/or a magnetic connection.
15. System according to any one of the preceding aspects, wherein the first component and/or the second component further comprise(s) at least one stop device configured to selectively block and/or limit rotation of the first cable winch and/or the second cable winch.
16. System according to aspect 15, wherein the stop device is configured to be activated automatically, preferably when the first cable winch and the second cable winch are decoupled from each other, and/or manually.
17. System according to aspect 15 or 16, wherein the stop device is configured to engage with the first engagement device and/or the second engagement device.
18. System according to any one of aspects 15 to 17, wherein the stop device is configured to be deactivated automatically, preferably when the first cable winch and the second cable winch are coupled to each other, and/or manually.
19. System according to any one of aspects 15 to 18, wherein the stop device includes one or more teeth configured to engage one or more teeth of the first cable winch and/or one or more teeth of the second cable winch, wherein engagement between the teeth of the stop device and the teeth of the first cable winch and/or the second cable winch blocks and/or limits rotation of the first cable winch and/or the second cable winch.
20. System according to any one of aspects 15 to 19, wherein the stop device is biased towards an engaged position in which the stop device blocks and/or limits rotation of the first cable winch and/or the second cable winch.
21. System according to aspect 20, wherein the stop device includes at least one spring element, preferably at least one spring steel sheet, configured to bias the stop device towards the engaged position.
22. System according to aspect 20 or 21, wherein the stop device is configured such that at least a portion of the stop device is deflected away from the engaged position, when the first cable winch and the second cable winch are coupled to each other, to a disengaged position, in which the stop device does not block and/or limit rotation of the first cable winch and/or the second cable winch.
23. System according to any one of the preceding aspects, wherein the first component and the second component are mirror-symmetrical.
24. Assisting device configured to be worn by a user and to assist movement of the user, wherein the assisting device includes at least one system according to any one of the preceding aspects to transmit power from at least one power source, preferably at least one electric motor, to the assisting device.
25. Assisting device according to aspect 24, wherein the assisting device is configured as an orthosis, preferably a hand orthosis, or a prosthesis.
26. Assisting device according to aspect 24 or 25, wherein the assisting device is configured as a hand orthosis or a hand prosthesis, wherein the hand orthosis or hand prosthesis includes at least two finger elements, preferably at least two flexible finger elements, wherein the finger elements are coupled or couplable to the first cable and/or the second cable of the system to manipulate, preferably drive, the finger elements via the first cable and/or the second cable.
27. Assisting device according to any one of aspects 24 to 26, wherein the assisting device is configured to be adjustable in size.
28. Assisting device according to any one of aspects 24 to 27, wherein the assisting device includes at least two magnets configured to fixate the assisting device on the user's body.
29. Assisting device according to any one of aspects 24 to 28, wherein the assisting device includes at least one connecting device, preferably at least one snap fastener and/or at least one Velcro fastener, configured to fixate the assisting device to the user's body.
30. Assisting device according to any one of aspects 24 to 29, wherein the assisting device includes the at least one power source.
31. Robot configured to be manipulated about and/or along one or more axes, wherein the robot includes at least one system according to any one of aspects 1 to 23 to transmit power from at least one power source, preferably at least one electric motor, to the robot.
32. Method of operating, a system for transmitting power, preferably the system according to any one of aspects 1 to 23, the system comprising:
   a first component configured to at least partially receive a first transmitting device, preferably a first cable, wherein the first component comprises a first engagement device, preferably wherein the first component includes a first cable winch configured to rotate to at least partially wind and unwind the first cable on the first cable winch;
   a second component configured to at least partially receive a second transmitting device, preferably a second cable, wherein the second component comprises a second engagement device configured to engage with the first engagement device, preferably wherein the second component includes a second cable winch configured to rotate to at least partially wind and unwind the second cable on the second cable winch;
   the method comprising:
      engaging the first engagement device with the second engagement device to couple the first cable, preferably the first cable winch, and the second cable, preferably the second cable winch, to each other, preferably such that rotation of one of the first cable winch and the second cable winch causes rotation of the other of the first cable winch and the second cable winch, when the first cable winch and the second cable winch are in a coupled state; and/or
      disengaging the first engagement device from the second engagement device to decouple the first cable, preferably the first cable winch, from the second cable, preferably the second cable winch.
33. Method according to aspect 32, comprising:
   activating at least one stop device by moving the stop device to an engaged position to block and/or limit movement, preferably rotation, of the first cable, preferably the first cable winch, and/or the second cable, preferably the second cable winch, by the stop device.
34. Method according to aspect 33, wherein the stop device is moved automatically to the engaged position when the first cable, preferably the first cable winch, and the second cable, preferably the second cable winch, are decoupled from each other.
35. Method according to aspect 33 or 34, comprising:
   deactivating the stop device by moving the stop device from the engaged position to a disengaged position, in which the stop device does not block and/or limit movement, preferably rotation, of the first cable, preferably the first cable winch, and/or the second cable, preferably the second cable winch, preferably automatically, preferably when the first cable, preferably the first cable winch, and the second cable, preferably the second cable winch, are coupled to each other.

Preferred embodiments of the present invention are further elucidated below with reference to the figures. The described embodiments are merely exemplary and do not limit the present invention, as defined by the claims and their respective equivalents.
- Fig. 1: shows, in a schematic top view, a system for transmitting power according to an embodiment of the present disclosure, the system being in a decoupled state;
- Fig. 2: shows, in a schematic and cross-sectional view, the system of Fig. 1;
- Fig. 3: shows, in a schematic top view, the system of Fig. 1 in a coupled state;
- Fig. 4: shows, in a schematic and cross-sectional view, the system of Fig. 3;
- Fig. 5: shows, in a schematic side view, the system of Figs. 3 and 4;
- Fig. 6: shows, in a schematic exploded view, the first component of the system of Figs. 1 to 5;
- Fig. 7: shows, in a schematic exploded view, the second component of the system of Figs. 1 to 5;
- Fig. 8: shows, in a schematic perspective view, an assisting device which includes at least one system of Figs. 1 to 5.

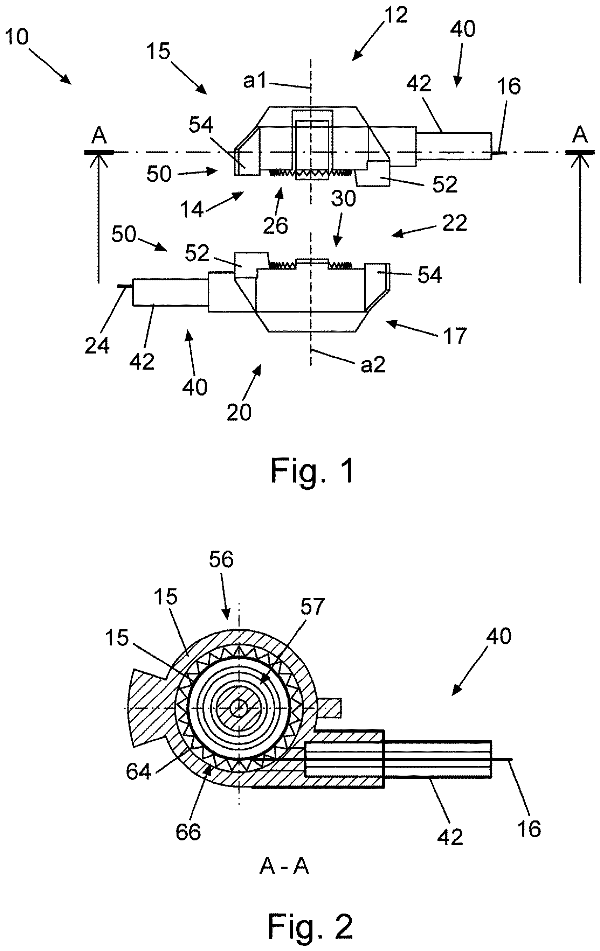

Fig. 1 shows, in a schematic illustration, a top view of a system 10 for transmitting power. The system 10 may comprise a 2first component 12 which may include a first cable winch 14 configured to rotate to at least partially wind and unwind a first cable 16 on the first cable winch 14. The system 10 may comprise a second component 20 which includes a second cable winch 22 configured to rotate to at least partially wind and unwind a second cable 24 on the second cable winch 22.

The first component 12 may comprise a first engagement device 26 and the second component 20 comprises a second engagement device 30 configured to selectively engage with the first engagement device 26 to operatively couple and decouple the first cable winch 14 and the second cable winch 22 to and from each other, respectively, such that rotation of one of the first cable winch 14 and the second cable winch 22 causes rotation of the other of the first cable winch 14 and the second cable winch 22, when the first cable winch 14 and the second cable winch 22 are in a coupled state.

The first engagement device 26 and the second engagement device 30 may be any engagement device(s) which allow(s) the first engagement device 26 and the second engagement device 30 to be operatively coupled and decoupled and to transmit forces and/or torque between the first cable winch 14 and the second cable winch 22 in a coupled state. The first engagement device 26 may be configured as a first toothed gearing and the second engagement device 30 may be configured as a second toothed gearing. The first toothed gearing and the second toothed gearing may each configured as a face gearing or a bevel gearing. Alternatively, different mechanisms may be employed for coupling and decoupling the first cable winch 14 and the second cable winch 22 to and from each other, respectively. For instance, the first engagement device 26 and the second engagement device 30 may be configured as a friction clutch, i.e., such that the first engagement device 26 may be configured to frictionally engage the second engagement device 30 such that rotational movement may be transferred between the first cable winch 14 and the second cable winch 22 via friction (e.g., only friction) between the first engagement device 26 and the second engagement device 30. Further alternatively, as a further example, the first engagement device 26 and the second engagement device 30 may be configured as a tongue and groove connection.

The first component 12 may include a housing 15 configured to at least partially house the first cable 16 and/or the first cable winch 14. Alternatively, or additionally, the second component 20 may include a housing 17 configured to at least partially house the second cable 24 and/or the second cable winch 22.

The system 10 may be configured such that, when the first cable winch 14 and the second cable winch 22 are in a coupled state, rotation of the first cable winch 14 rotatably drives the second cable winch 22 such that the first cable 16 is wound onto the first cable winch 14 and the second cable 24 is unwound from the second cable winch 22 and/or the first cable 16 is unwound from the first cable winch 14 and the second cable 24 is wound onto the second cable winch 22. Alternatively, or additionally, the system 10 may be configured such that, when the first cable winch 14 and the second cable winch 22 are in a coupled state, rotation of the second cable winch 22 rotatably drives the first cable winch 14 such that the first cable 16 is wound onto the first cable winch 14 and the second cable 24 is unwound from the second cable winch 22 and/or the first cable 16 is unwound from the first cable winch 14 and the second cable 24 is wound onto the second cable winch 22.

The system 10 may be configured such that a winding axis a1 of the first cable winch 14 and a winding axis a2 of the second cable winch 22 substantially coincide.

The first cable winch 14 may be biased towards winding the first cable 16 onto the first cable winch 14. Alternatively, or additionally, the second cable winch 22 may be biased towards winding the second cable 24 onto the second cable winch 22.

The system 10 may include at least one Bowden cable 40 which may include at least one cable sheath 42 in which the first cable 16 and/or the second cable 24 is/are guidable such that the first cable 16 and/or the second cable 24 is/are moveable relative to the at least one cable sheath 42.

The system 10 may include at least one securing device 50 configured to fixedly secure the first component 12 to the second component 20, e.g., to maintain the first cable winch 14 and the second cable winch 22 in a coupled state. The securing device 50 may be configured as a quick-release mechanism. For instance, the securing device 50 may be configured as a snap-fit connection, a screw connection, and/or a magnetic connection.

The securing device 50 may include at least one first securing element 52 provided and/or defined on the first component 12 and at least one second securing element 54 provided and/or defined on the second component 20. The first securing element 52 and the second securing element 54 may be configured to releasably engage each other to fixedly secure the first component 12 to the second component 20. For instance, the first securing element 52 and/or the second securing element 54 may be configured as a clip and/or latch configured to engage the other of first securing element 52 and the second securing element 54. The first securing element 52 and the second securing element 54 may be configured to releasably engage in a frictional and/or form-fit manner.

The securing elements 52, 54 may be defined on the housing 15 of the first component 12 and/or the housing 17 of the second component 20.

Fig. 2 shows a schematic cross-sectional view of the system 10 of Fig. 1 along the line A-A indicated in Fig. 1. As shown in Fig. 2, the first cable winch 14 and/or the second cable winch 22 may include at least one rotary bearing 57 for enabling rotation of the first cable winch 14 and/or the second cable winch 22, e.g., relative to the housing 15 and 17, respectively. The rotary bearing 57 may be configured as a rolling bearing, preferably at least one ball bearing.

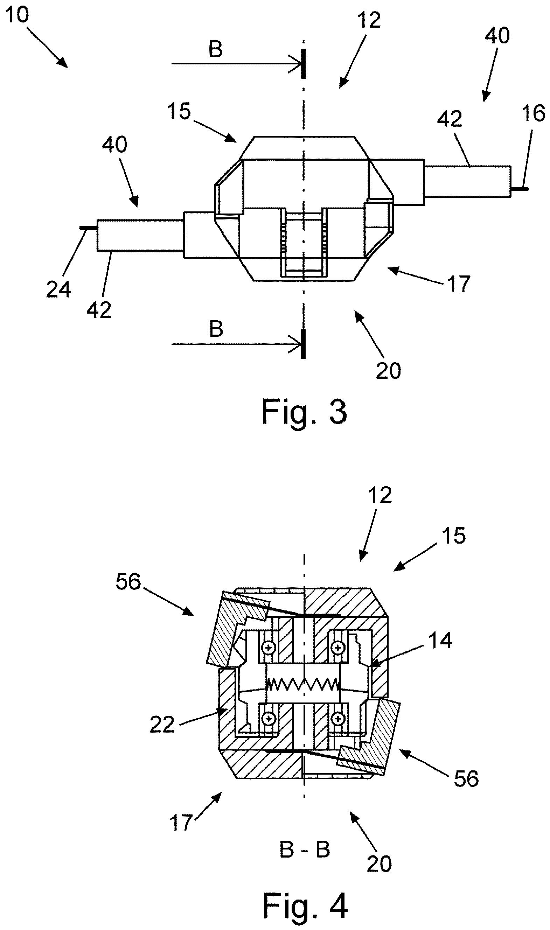

Figs. 1 and 2 show the system 10 in a decoupled state, i.e., in which the first component 12 and the first component 20, more specifically the first engagement device 26 and the second engagement device 30, are decoupled from each other. By contrast, Figs. 3 to 5 show the system 10 in a coupled state, i.e., in which the first component 12 and the first component 20, more specifically the first engagement device 26 and the second engagement device 30, are coupled/engaged to each other. Fig. 4 shows a schematic cross-sectional view of the system 10 along the line B-B indicated in Fig. 3.

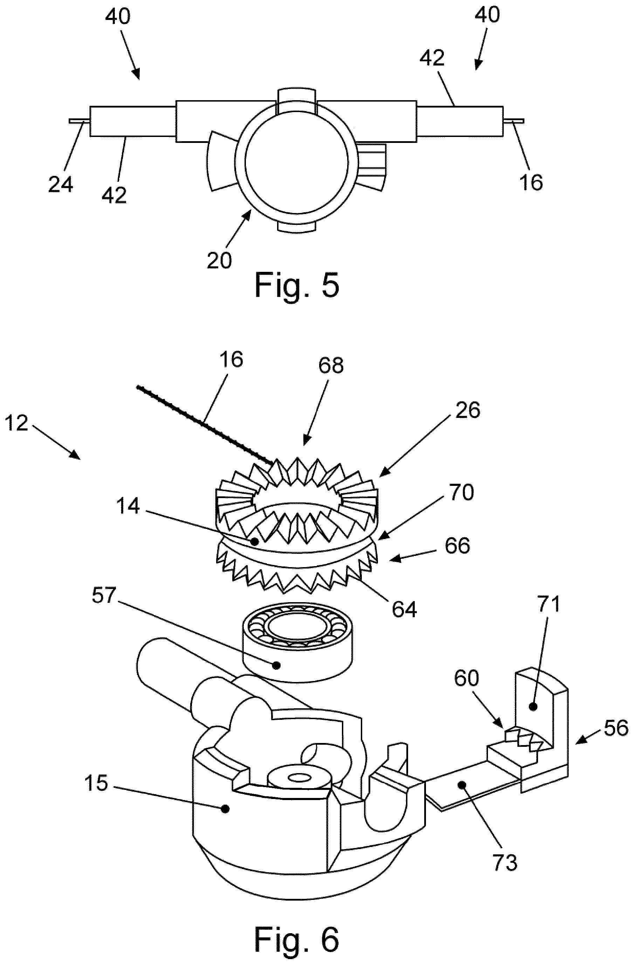

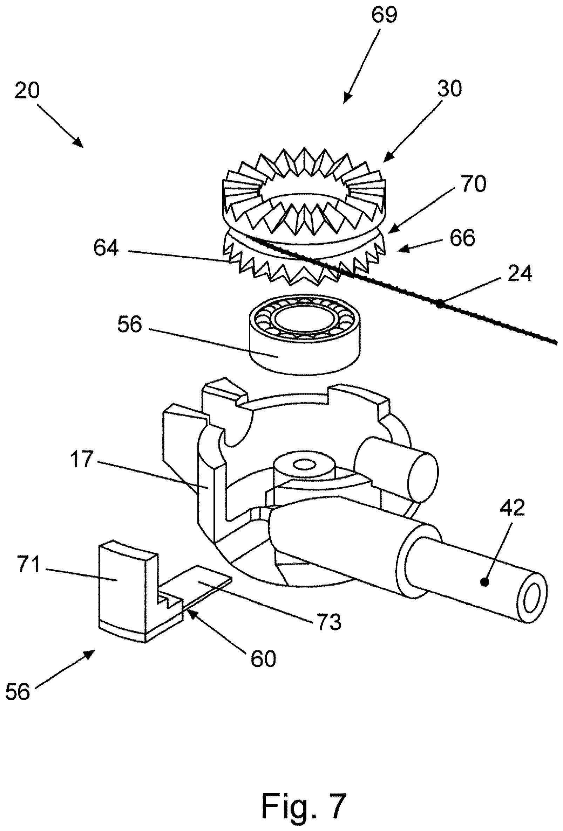

The first component 12 (see Fig. 6) and/or the second component 20 (see Fig. 7) may comprise at least one stop device 56 (as best seen in Figs. 6 and 7) configured to selectively block and/or limit rotation of the first cable winch 14 and/or the second cable winch 22, when the stop device 56 is in an engaged position. As shown in Figs. 6 and 7, the first component 12 and the second component 20 may each include a stop device 56. However, for the sake of clarity, the stop device 56 will only be described based on the first component 12 shown in Fig. 6. The same applies to the second component 20 of Fig. 7.

The stop device 56 may be configured to be deactivated automatically, preferably when the first cable winch 14 and the second cable winch 22 are coupled to each other, and/or manually.

The stop device 56 may include at least one engaging component 58 which may include one or more teeth 60 configured to engage one or more teeth 64 of the first cable winch 14 and/or one or more teeth 64 of the second cable winch 22. Engagement between the teeth 60 of the stop device 56 and the teeth 64 of the first cable winch 14 and/or the second cable winch 22 may block and/or limit rotation of the first cable winch 14 and/or the second cable winch 22. The teeth 64 of the first cable winch 14 and/or the second cable winch 22 may be provided and/or defined on a circumference of the first cable winch 14 and/or the second cable winch 22. For instance, the first cable winch 14 and/or the second cable winch 22 may include at least one wheel 66 on which the teeth 64 are provided and/or defined. The wheel 66 may be included by a rotating unit 68 (rotating unit 69 in the case of the second component 20) which may include the wheel 66, the first engagement device 26, which is configured as a toothed gearing, particularly a face gearing in Fig. 6, and a cable receiving portion 70 configured to receive at least a section of the cable 16 by winding the cable 16 at least partially about the cable receiving portion 70.

The stop device 56 may include a main structure 71 on which the teeth 60 are defined. The stop device 56 may be biased towards the engaged position in which the stop device 56 blocks and/or limits rotation of the first cable winch 14 and/or the second cable winch 22. In particular, the bias, e.g., one or more biasing forces toward the engaged position, may be provided, e.g., by at least one spring element 73. For instance, at least one spring element 73, e.g., spring steel sheet, may be provided. Alternatively, or additionally, the stop device 56 may be shaped and/or formed and/or dimensioned such that the stop device 56 may be biased towards the engaged position, i.e., such that the stop device 56 is in the engaged position, in the absence of any counter-forces, or when one or more counter-forces are below a certain threshold.

The stop device 56 may be configured such that at least a portion of the stop device 56 is deflected away from the engaged position, when the first cable winch 14 and the second cable winch 22 are coupled to each other, to a disengaged position, in which the stop device 56 does not block and/or limit rotation of the first cable winch 14 and/or the second cable winch 22.

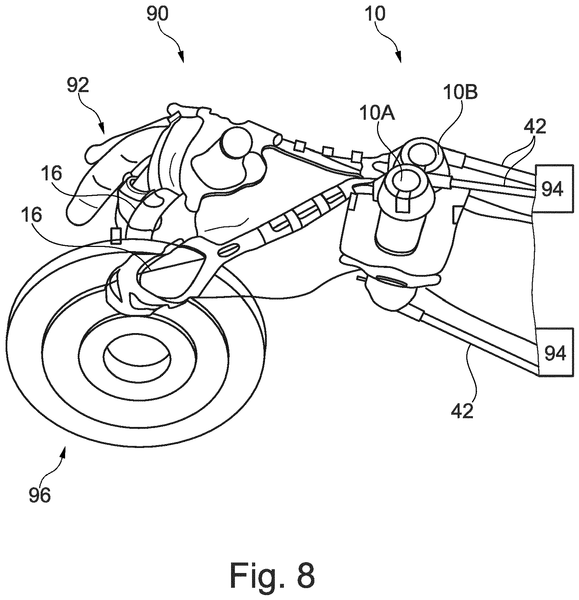

Fig. 8 shows an assisting device 90 configured to be worn by a user, in particular at least partially on a user's hand 92. The assisting device 90 may be configured to assist movement and/or static actions, such as grasping an object 96, of the user. The assisting device 90 may include at least one system 10 according to any one of the embodiments described herein to transmit power from at least one power source 94, preferably at least one electric motor, to the assisting device 90.

The assisting device 90 may be configured as an orthosis, preferably a hand orthosis, or a prosthesis, preferably a hand prosthesis.

The location and configuration of the power source 94 is shown only schematically and exemplarily in Fig. 8. The power source 94 may be arranged and/or arrangeable at various positions, preferably on the user's body.

The assisting device 90 may be configured to assist the user in grasping and/or moving the object 96.

The assisting device 90 may include a single system 10 or a plurality of systems 10, as shown in Fig. 8. For instance, at least two systems 10A and 10B may be provided to individually assist movement of a respective finger of the user's hand 92.

The first cable 16 or the second cable 24 may be guided at least partially along and attached to the respective finger. As an example, the first cable 16 is depicted in Fig. 8 as being guided at least partially along and attached to the respective finger. The second cable 24 may be guided at least partially within the sheath 42, in particular to provide a Bowden cable.

## Claims

1. System (10) for transmitting power, the system (10) comprising:
a first component (12) which includes a first cable winch (14) configured to rotate to at least partially wind and unwind a first cable (16) on the first cable winch (14);
a second component (20) which includes a second cable winch (22) configured to rotate to at least partially wind and unwind a second cable (24) on the second cable winch (22);
wherein the first component (14) comprises a first engagement device (26) and the second component (20) comprises a second engagement device (30) configured to selectively engage with the first engagement device (20) to couple and decouple the first cable winch (14) and the second cable winch (22) to and from each other, respectively, such that rotation of one of the first cable winch (14) and the second cable winch (22) causes rotation of the other of the first cable winch (14) and the second cable winch (22), when the first cable winch (14) and the second cable winch (22) are in a coupled state.

2. System (10) according to claim 1, wherein the system is configured such that, when the first cable winch (14) and the second cable winch (22) are in a coupled state:
rotation of the first cable winch (14) rotatably drives the second cable winch (22) such that the first cable (16) is wound onto the first cable winch (14) and the second cable (24) is unwound from the second cable winch (22) and/or the first cable (16) is unwound from the first cable winch (14) and the second cable (24) is wound onto the second cable winch (22);
and/or
rotation of the second cable winch (22) rotatably drives the first cable winch (14) such that the first cable (16) is wound onto the first cable winch (14) and the second cable (24) is unwound from the second cable winch (22) and/or the first cable (16) is unwound from the first cable winch (14) and the second cable (24) is wound onto the second cable winch (22).

3. System (10) according to any one of the preceding claims, wherein a winding axis (a1) of the first cable winch (14) and a winding axis (a2) of the second cable winch (22) coincide.

4. System (10) according to any one of the preceding claims, wherein the first cable winch (14) is biased towards winding the first cable (16) onto the first cable winch (14) and/or the second cable winch (22) is biased towards winding the second cable (24) onto the second cable winch (22).

5. System (10) according to any one of the preceding claims, wherein the first engagement device (26) is configured as a first toothed gearing and the second engagement device (30) is configured as a second toothed gearing.

6. System according to claim 5, wherein the first toothed gearing and the second toothed gearing are each configured as a face gearing or a bevel gearing.

7. System (10) according to any one of the preceding claims, further including at least one Bowden cable (40) which includes at least one cable sheath (42) in which the first cable (16) and/or the second cable (24) is/are guidable such that the first cable (16) and/or the second cable (24) is/are moveable relative to the at least one cable sheath (42).

8. System (10) according to any one of the preceding claims, further including at least one securing device (50) configured to fixedly secure the first component (12) to the second component (20) to maintain the first cable winch (14) and the second cable winch (22) in a coupled state, wherein the securing device (50) is configured as a quick-release mechanism.

9. System (10) according to any one of the preceding claims, wherein the first component (12) and/or the second component (20) further comprise(s) at least one stop device (56) configured to selectively block and/or limit rotation of the first cable winch (14) and/or the second cable winch (22), when the stop device (56) is in an engaged position.

10. System (10) according to claim 9, wherein the stop device (56) is configured to be deactivated automatically, preferably when the first cable winch (14) and the second cable winch (22) are coupled to each other, and/or manually.

11. System (10) according to claim 9 or 10, wherein the stop device (56) includes one or more teeth (60) configured to engage one or more teeth (64) of the first cable winch (14) and/or one or more teeth (64) of the second cable winch (22), wherein engagement between the teeth (60) of the stop device (56) and the teeth (64) of the first cable winch (14) and/or the second cable winch (22) blocks and/or limits rotation of the first cable winch (14) and/or the second cable winch (22).

12. System (10) according to any one of claims 9 to 11, wherein the stop device (56) is biased towards the engaged position in which the stop device (56) blocks and/or limits rotation of the first cable winch (14) and/or the second cable winch (22).

13. System (10) according to any one of claims 9 to 12, wherein the stop device (56) is configured such that at least a portion of the stop device (56) is deflected away from the engaged position, when the first cable winch (14) and the second cable winch (22) are coupled to each other, to a disengaged position, in which the stop device (56) does not block and/or limit rotation of the first cable winch (14) and/or the second cable winch (22).

14. Assisting device (90) configured to be worn by a user and to assist movement of the user and/or grasping and/or moving an object by the user, wherein the assisting device (90) includes at least one system (10) according to any one of the preceding claims to transmit power from at least one power source (94), preferably at least one electric motor, to the assisting device (90), preferably wherein the assisting device (90) is configured as an orthosis, preferably a hand orthosis, or a prosthesis.

15. Method of operating a system (10) for transmitting power, preferably the system (10) according to any one of claims 1 to 13, the system (10) comprising:
a first component (12) which includes a first cable winch (14), which is configured to rotate to at least partially wind and unwind a first cable (16) on the first cable winch (14), and a first engagement device (26);
a second component (20) which includes a second cable winch (22), which is configured to rotate to at least partially wind and unwind a second cable (24) on the second cable winch (22), and a second engagement device (30) configured to selectively engage with the first engagement device (20);
the method comprising:
engaging the first engagement device (20) with the second engagement device (30) to couple the first cable winch (14) and the second cable winch (22) to each other such that rotation of one of the first cable winch (14) and the second cable winch (22) causes rotation of the other of the first cable winch (14) and the second cable winch (22), when the first cable winch (14) and the second cable winch (22) are in a coupled state;
and/or
disengaging the first engagement device (20) from the second engagement device (30) to decouple the first cable winch (14) from the second cable winch (22).
